# EUROPEAN PATENT SPECIFICATION

(11) **EP 0 403 631 B1**
(45) Date of publication and mention of the grant of the patent: **09.08.1995**
(21) Application number: 90901355.9
(22) Date of filing: 15.12.1989
(51) Int. Cl.: H01L 21/60

(54) **METHOD OF MAKING HIGH DENSITY SOLDER BUMPS AND A SUBSTRATE SOCKET FOR HIGH DENSITY SOLDER BUMPS**
VERFAHREN ZUM HERSTELLEN VON LÖTHÖCKERN HOHER DICHTE UND EIN SUBSTRATSOCKEL FÜR LÖTHÖCKER HOHER DICHTE
PROCEDE POUR REALISER DES PERLES DE BRASURE A HAUTE DENSITE, ET SOCLE DE SUBSTRAT POUR CELLES-CI

(30) Priority: 03.01.1989 US 292988
(43) Date of publication of application: 27.12.1990
(73) Proprietor: MOTOROLA, INC., Schaumburg, IL 60196 (US)
(72) Inventor: ALTMAN, Leonard, F., Coral Springs, FL 33065 (US); FLAUGHER, Jill, L., Margate, FL 33063 (US); SUPPELSA, Anthony, B., Coral Springs, FL 33065 (US); MULLEN, William, B., III, Boca Raton, FL 33428 (US)
(74) Representative: Ibbotson, Harold
(86) International application number: US8905528
(87) International publication number: WO9007792

(56) References cited:
- EP-A- 0 270 067
- JP-A- 0 136 990
- US-A- 3 436 818
- US-A- 3 589 000
- US-A- 3 986 255
- US-A- 4 032 058
- US-A- 4 273 859
- US-A- 4 311 267
- US-A- 4 739 917
- US-A- 4 818 728
- IBM Technical Disclosure Bulletin, Vol. 15, No. 5, p. 1715, October 1972
- IBM Technical Disclosure Bulletin, Vol. 17, No. 8, p. 2331, January 1975
- Metals Handbook Ninth Edition, Vol.6, pp. 1100, 1101, copyright 1983

## Description

### BACKGROUND OF THE INVENTION:

This invention relates to solder bumps in general and particularly to a method for making high density solder bumps and for providing a socket on a substrate for receiving a solder bumped member. The use of solder bumps on a substrate for attachment to a second substrate by a reflow process is well known in the art. Solder bumps are particularly useful with pad grid array substrates. The closeness or density of solder pads is limited using known technologies due to problems in providing high density solder bumps and preventing the solder bumps from flowing together and shorting pads and bumps when reflowed. One approach for providing solder bumps involves using a stencil process where a stencil is placed over the substrate and solder paste is applied to the substrate through the stencil as by using a squeegee. With this approach the density is limited due to the slumping of the solder paste when the stencil is removed.

Another approach involves the use of solder balls which are precisely located on a substrate. The placing of solder balls is time consuming and can present reliability problems. If a single ball is misplaced, the proper soldering of the substrate will not be accomplished.

Another concept has been described by Mones et al. in U.S. Patent 4,273,859, where an improved method of forming raised input/output terminals on the top surface of a semiconductor chip is disclosed. A photoresist layer is deposited on the chip, and appropriate openings are defined over the selected terminals. A layer of solder is then deposited on the entire chip, covering the exposed terminals and the photoresist. After reflowing the solder, the excess solder on the resist is removed by lifting off the resist. Japanese Patent JP-A-136990 and the *Metals Handbook, Ninth Edition*, Volume 6, pages 1100-1101 describe uses of solder paste in electronic applications. However, these methods suffer from the drawback of having to remove the excess solder deposited on the photoresist, along with the requirement of having to remove all of the photoresist.

### SUMMARY OF THE INVENTION

In accordance with a first aspect of the invention there is provided a method of forming solder bumps comprising the steps of: applying a thick layer of resist on a substrate; forming wells in the resist by selectively removing first portions thereof over solder pads on the substrate thereby leaving second portions of the resist on the substrate; applying solder paste only in the wells to contact the solder pads; and reflowing the solder paste to form solder bumps on the solder pads; wherein the resist second portions remaining on the substrate during the step of reflowing. In a second aspect of the invention there is provided a method of attaching a solder bumped member to a substrate, comprising the steps of: providing a substrate having metallized pads corresponding to the solder bumps of the member; applying a thick layer of resist to the substrate; selectively removing the resist to provide wells at said metallized pads on the substrate; depositing solder paste in the wells with a squeegee; positioning the member so that the solder bumps are in the wells; and heating the solder paste to reflow and bond to the solder bumps and the metallized pads.

These methods of providing high density solder bumps permit close spacing of the solder and a reliable technique for solder application. Also, these methods of attaching a solder bumped member to a substrate provide a socket for self-alignment of the solder bumped member.

In one aspect of the invention, the solder paste is selected to have a melting temperature less than that of the solder bumps. During the heating step, the solder paste is heated to a temperature less than the melting temperature of the solder bumps, but sufficiently high to melt the solder paste and to effect a metallurgical connection to both the higher melting temperature solder bumps and the metallized pads of the substrate.

An exemplary embodiment of the present invention will now be described with reference to the accompanying drawings.

### BRIEF DESCRIPTION OF THE DRAWINGS

FIG. 1 is a top plan view of an array of substrates with metallized pads.

FIG. 2 is a top plan view of the array of substrates after application of solder paste.

FIG. 3 is a cross-sectional view of a substrate covered with undefined solder resist.

FIG. 4 is a cross-sectional view of the substrate of FIG. 3 after selective delineation and removal of resist.

FIG. 5 is a cross-sectional view of the substrate of FIG. 3 after application of solder paste.

FIG. 6 is a cross-sectional view of the substrate of FIG. 3 after reflow of the paste.

FIG. 7 is a cross-sectional view of the substrate of FIG. 3 after removal of the remaining resist.

FIG. 8 is a cross-sectional view of a substrate that can be used as a socket for a solder bumped member, shown covered with undefined solder resist.

FIG. 9 is a cross-sectional view of the substrate of FIG. 8 after selective delineation and removal of resist.

FIG. 10 is a cross-sectional view of the substrate of FIG. 8 after application of solder paste.

FIG. 11 is a cross-sectional view of the substrate of FIG. 8 showing the positioned bumped member.

FIG. 12 is a cross-sectional view of the substrate of FIG. 8 after reflow of the solder paste.

### DESCRIPTION OF THE PREFERRED EMBODIMENT:

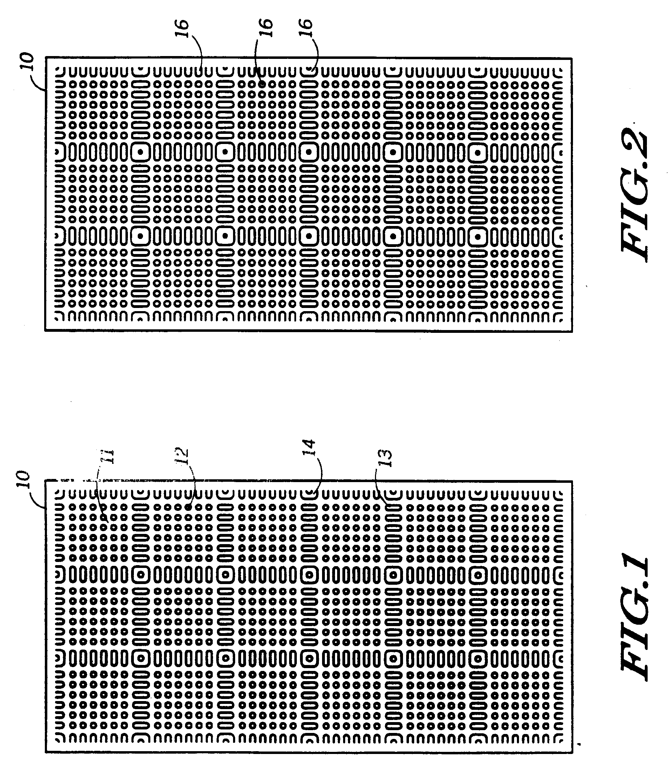

Referring first to FIG. 1, a substrate 10 is illustrated which includes fifteen pad grid arrays 11 on a single substrate. Each of the pad grid arrays 11 includes a plurality of metalized pads 12 arranged in a matrix. About the periphery of each pad grid array 11 is a plurality of side pads 13 and corner pads 14. The substrate 10 can be any desired material as for example ceramic. The individual pad grid arrays when separated can be used for electronic components such as for integrated circuit chip carriers.

FIG. 2, illustrates the substrate 10 after the application of photo definable solder resist which has been exposed and processed to provide areas about the metalized pads 12, 13, and 14 which are slightly larger than the area of the metalized pads. These areas have been filled with solder paste 16 as is discussed below.

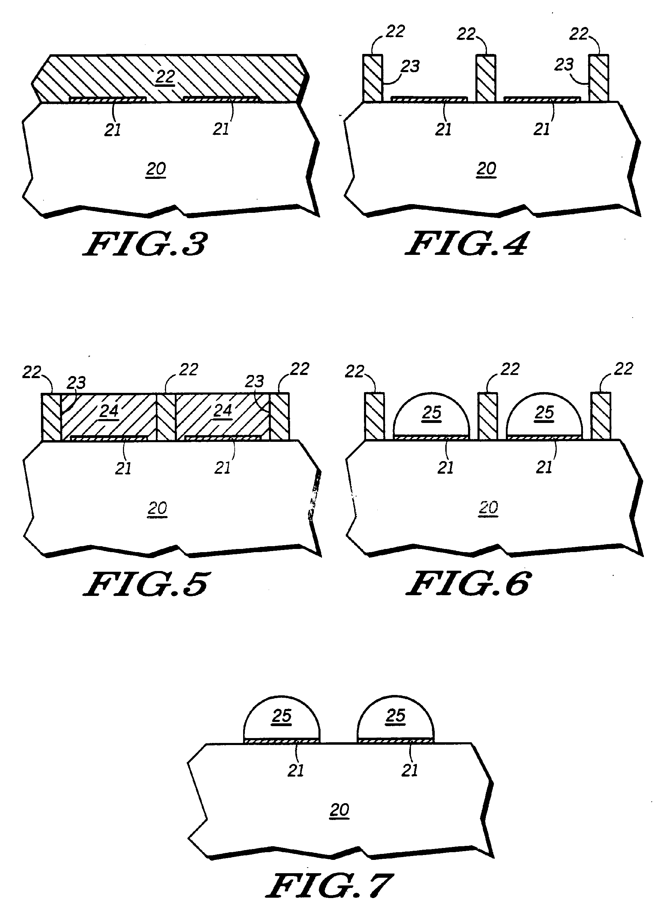

FIG. 3 illustrates a substrate 20 which for clarity shows only two metalized pads 21 which can include a thin layer of solder. The substrate 20 can include any desired pattern of metalized pads 21, which can include a thin layer of solder. A thick layer of photo definable solder resist 22 is applied to the substrate 20. The resist 22 is preferably at least 0.254 mm (10 mils.) thick and preferably 0.38 mm (15 mils.) thick. Suitable photo definable solder resist is manufactured by Armstrong World Industries of Lancaster, Pennsylvania and sold under the trademarks FANTON 106, 306, 370, or 363. The resist 22 is then exposed with ultraviolet radiation through a master stencil which defines the areas over the metalized pads 21 and can define an area larger than the metalized pads 21, by sensitizing the resist to then allow selective removal of the resist.

The selective removal of the resist 22 provides wells 23 as illustrated in FIG. 4. Solder paste 24 is then applied directly to the substrate 20 as by a squeegee. The remaining resist 22 acts essentially as a stencil capturing the solder paste 24 in the wells 23, as illustrated in FIG. 5. The size of the wells 23 is selected so that an appropriate amount of solder paste 24 is captured in each well. The solder paste 24 is then heated to reflow the solder and form the solder bumps 25 illustrated in FIG. 6. The height of the solder bumps 25 is determined by the composition of the solder paste 24, the area of the metalized pad 21, and by the dimensions of the well 23. Where higher solder bumps 25 are desired, larger wells 23 can be provided so that more solder paste 24 is available for reflow. The solder paste 24, when reflowed, wets and wicks to the metalized pads 21. After the reflow operation, the remaining solder resist 22 can be stripped or removed from the substrate 20 to provide the final solder bumped substrate as illustrated in FIG. 7.

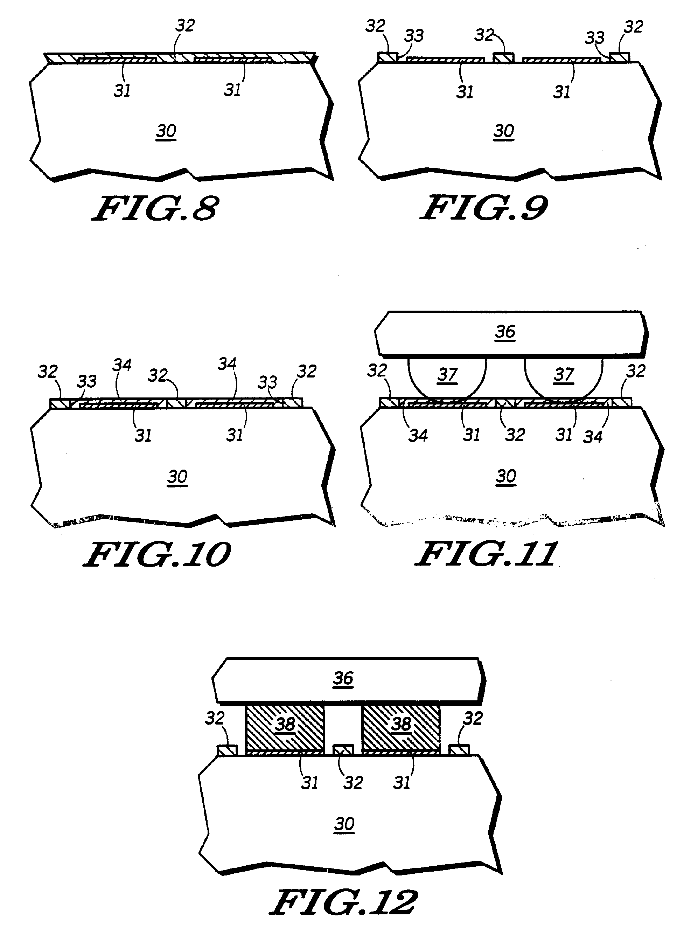

A similar approach can be utilized for providing a mating substrate for attachment of a solder bumped substrate. A self-fixturing substrate is provided by substrate 30 of FIG. 8. The substrate 30 can be ceramic, polyimide, a printed circuit board or other substrate. The substrate 30 includes metalized pads 31 having a pattern corresponding to that of the solder bumped substrate to which it is being connected, such as that of FIG. 7.

A photo definable solder resist 32 (which can be the same as resist 22) is applied over the substrate 30 and is delineated to define wells 33 in the photoresist 32, as illustrated in FIG. 9. Solder paste 34 is then directly applied, as by a squeegee, to fill the wells 33 as illustrated in FIG. 10. A solder bumped member 36 having solder bumps 37, which can be the solder bumped substrate 20 of FIG. 7, is positioned over the substrate 30 so that its solder bumps 37 settle into the solder paste 34. The wells 33 are preferably about 0.254 mm (10 mils.) (0.01 inches) high and provide the self-aligning or fixturing of the solder bumped member 36, The solder bumps 37 tend to settle into the solder paste 34 under the force of gravity . The assembly is then heated to reflow the solder paste 34 for bonding with the solder bumps 37 and interconnecting the member 36 to substrate 30. A resulting solder interconnection is illustrated as solder 38 of FIG. 12.

Preferably, the solder paste 34 is selected to have a lower melting temperature than the solder of the solder bumps 37. By heating the solder paste 34 to a temperature sufficient to reflow it but less than the melting temperature of the solder bumps 37, the solder paste 34 will flow and wet to the metalized pads 31 and the solder bumps 37, blending with the solder bumps 37. While the solder bumps 37 can be reflowed, it is desirable not to reflow them in order to avoid the possibility of the inadvertent shorting of adjacent solder bumps.

## Claims

1. A method of forming solder bumps comprising the steps of:
applying a thick layer of resist (19) on a substrate (20);
forming wells (23) in the resist by selectively removing first portions thereof over solder pads (21) on the substrate thereby leaving second portions (22) of the resist on the substrate;
applying solder paste (24) only in the wells (23) to contact the solder pads (21); and
reflowing the solder paste (24) to form solder bumps (25) on the solder pads;
wherein the resist second portions (22) remaining on the substrate (20) during the step of reflowing.

2. A method of forming solder bumps in accordance with claim 1, including the further step of removing the remaining resist (22) after forming the solder bumps (24).

3. A method of forming solder bumps in accordance with claim 1 or 2, in which the solder paste (24) is applied to the substrate (20) with a squeegee.

4. A method of forming solder bumps in accordance with claim 1, 2 or 3, in which the wells (23) are formed to be slightly larger than the solder pads (21).

5. A method of forming solder bumps as in accordance with any preceding claim, in which the second portions of resist (22) remain on the substrate (20) permanently.

6. A method of forming solder bumps as in accordance with any preceding claim, in which the layer of resist (19) is approximately 0.4 mm thick.

7. A method of attaching a solder bumped member to a substrate, comprising the steps of:
providing a substrate (30) having metallized pads (31) corresponding to the solder bumps (37) of the member (36);
applying a thick layer of resist (32) to the substrate;
selectively removing the resist to provide wells (33) at said metallized pads on the substrate;
depositing solder paste (34) in the wells with a squeegee;
positioning the member so that the solder bumps are in the wells; and
heating the solder paste to reflow and bond to the solder bumps and the metallized pads.

8. A method of attaching a solder bumped member to a substrate as defined in claim 7, wherein:
said solder paste (34) is selected to have a melting temperature less than that of the solder bumps (37); and
during the heating step, the solder paste is heated to a temperature less than the melting temperature of the solder bumps.

## Patentansprüche

1. Verfahren zur Bildung von Löthöckern, das die Schritte umfaßt:
Auftragen einer dicken Schicht von Resist (19) auf ein Substrat (20);
Bilden von Wannen (23) in dem Resist, indem erste Teile davon über Lötflecken (21) auf dem Substrat selektiv entfernt werden, wodurch zweite Teile (22) des Resists auf dem Substrat belassen werden;
Auftragen von Lötpaste (24) nur in den Wannen (23), um die Lötflecken (21) zu berühren, und
Schmelzen der Lötpaste (24), um Löthöcker (25) auf den Lötflecken zu bilden,
wobei die zweiten Teile (22) des Resists während des Schrittes des Schmelzens auf dem Substrat (20) zurückbleiben.

2. Verfahren zur Bildung von Löthöckern nach Anspruch 1, das den weiteren Schritt des Entfernens des übrigen Resists (22) nach dem Bilden der Löthöcker (25) umfaßt.

3. Verfahren zur Bildung von Löthöckern nach Anspruch 1 oder 2, bei dem die Lötpaste (24) mit einem Quetscher auf das Substrat (20) aufgetragen wird.

4. Verfahren zur Bildung von Löthöckern nach Anspruch 1, 2 oder 3, bei dem die Wannen (23) so gebildet werden, daß sie etwas größer als die Lötflecken (21) sind.

5. Verfahren zur Bildung von Löthöckern nach einem der vorangehenden Ansprüche, bei dem die zweiten Teile des Resists (22) dauernd auf dem Substrat (20) verbleiben.

6. Verfahren zur Bildung von Löthöckern nach einem der vorangehenden Ansprüche, bei dem die Schicht von Resist (19) etwa 0.4 mm dick ist.

7. Verfahren zum Befestigen eines mit Löthöckern versehenen Elements an einem Substrat, das die Schritte umfaßt:
Bereitstellen eines Substrats (30) mit metallisierten Flecken (31), die den Löthöckern (37) des Elements (36) entsprechen;
Auftragen einer dicken Schicht von Resist (32) auf das Substrat;
selektives Entfernen des Resists, um Wannen (33) an den metallisierten Flecken auf dem Substrat zu bilden;
Ablagern von Lötpaste (34) in den Wannen mit einem Quetscher;
Positionieren des Elements, so daß sich die Löthöcker in den Wannen befinden, und
Erhitzen der Lötpaste, um zu schmelzen und sich mit den Löthöckern und den metallisierten Flecken zu verbinden.

8. Verfahren zum Befestigen eines mit Löthöckern versehenen Elements an einem Substrat nach Anspruch 7, bei dem:
die Lötpaste (34) so gewählt wird, daß sie eine niedrigere Schmelztemperatur als die Löthöcker (37) aufweist, und
während des Erhitzungschrittes die Lötpaste auf eine Temperatur erhitzt wird, die niedriger als Schmelztemperatur der Löthöcker ist.

## Revendications

1. Procédé pour former des perles de soudure, comprenant les étapes suivantes :
application d'une épaisse couche de résist (19) sur un substrat (20);
formation de puits (23) dans le résist par enlèvement sélectif de premières parties de ce résist au-dessus de plages de connexion à souder (21) sur le substrat, ce qui laisse des deuxièmes parties (22) du résist sur le substrat;
application de pâte à souder (24) uniquement dans les puits (23) pour entrée en contact avec les plages de connexion à souder (21); et
refusion de la pâte à souder (24) pour former des perles de soudure (25) sur les plages de connexion à souder;
dans laquelle les deuxièmes parties de résist (22) demeurent sur le substrat (20) pendant l'étape de refusion.

2. Procédé pour former des perles de soudure selon la revendication 1, comprenant l'étape supplémentaire consistant à retirer le résist restant (22) après la formation des perles de soudure (24).

3. Procédé pour former des perles de soudure selon la revendication 1 ou 2, dans laquelle la pâte à souder (24) est appliquée au substrat (20) avec une raclette.

4. Procédé pour former des perles de soudure selon la revendication 1, 2 ou 3, dans laquelle les puits (23) sont formés de façon à être légèrement plus grands que les plages de connexion à souder (21).

5. Procédé pour former des perles de soudure selon l'une quelconque des revendications précédentes, dans laquelle les deuxièmes parties de résist (22) demeurent en permanence sur le substrat (20).

6. Procédé pour former des perles de soudure selon l'une quelconque des revendications précédentes, dans laquelle l'épaisseur de la couche de résist (19) est approximativement égale à 0,4 mm.

7. Procédé de fixation d'un élément à perles de soudure sur un substrat, comprenant les étapes suivantes :
réalisation d'un substrat (30) ayant des plages de connexion métallisées (31) correspondant aux perles de soudure (37) de l'élément (36);
application d'une épaisse couche de résist (32) sur le substrat;
enlèvement sélectif du résist pour réaliser des puits (33) au niveau desdites plages de connexion métallisées sur le substrat;
dépôt de pâte à souder (34) dans les puits à l'aide d'une raclette;
positionnement de l'élément de façon à ce que les perles de soudure soient dans les puits; et
chauffage de la pâte à souder pour la refusion et la fixation aux perles à souder et aux plages de connexion métallisées.

8. Procédé de fixation d'un élément à perles de soudure sur un substrat suivant les indications de la revendication 7, dans laquelle :
ladite pâte à souder (34) est sélectionnée pour présenter une température de fusion inférieure à celle des perles à souder (37);
et, pendant l'étape de chauffage, la pâte à souder est chauffée à une température inférieure à la température de fusion des perles à souder.
